# EUROPEAN PATENT APPLICATION

(11) **EP 2 455 365 A1**
(43) Date of publication of application: **23.05.2012**
(21) Application number: 10192009.8
(22) Date of filing: 22.11.2010
(51) Int. Cl.: C07C 253/14

(54) **Process for the preparation cyano carboxylic acid esters**

(71) Applicant: Lonza Ltd, 4052 Basel (CH)
(72) Inventor: Litzmann, Oliver, 12203 Berlin (DE); Repke, Jens-Uwe, Prof., 12621 Berlin (DE); Lorenz, Hilke-Marie, 12045 Berlin (DE); Hanselmann, Paul, Dr., 3902 Brig-Glis (CH); Müller, Constanze, Dr. rer. nat., 4056 Basel (CH)
(74) Representative: Winter, Brandl, Fürniss, Hübner, Röss, Kaiser, Polte - Partnerschaft

(57) **Abstract**

The present invention relates to a process for the preparation of a cyano carboxylic acid ester of the formula 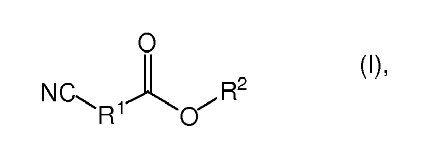 wherein
R¹ is a linear or branched C₁₋₈ alkanediyl group; and R² is a linear or branched alkyl group, a cycloalkyl group, or an aryl or arylalkyl group, wherein aryl is optionally substituted with one or more C₁₋₈ alkyl groups; comprising reacting a halo carboxylic acid ester of the formula 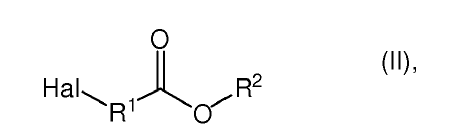 wherein R¹ and R² are as defined above; and Hal is fluorine, chlorine, bromine or iodine;
in a reaction mixture comprising a homogeneous liquid phase, wherein the liquid phase consists of water and an organic solvent,
with an alkali metal cyanide in the presence of hydrogen cyanide, optionally in the presence of a catalyst, to obtain the cyano carboxylic acid ester of the formula (I).

## Description

The present invention relates to a process for production of cyano carboxylic acid esters.

Cyano carboxylic acid esters such as cyano acetates are important intermediates in the synthesis of, e.g., vitamin B6, caffeine, folic acid and many other products such as cyano acrylates which are used in superglues.

Cyano carboxylic acid esters such as cyano acetates may be formed by reacting a corresponding halo carboxylic acid ester with alkali metal cyanide in a nucleophilic substitution reaction to form the cyano carboxylic acid ester and the corresponding alkali metal halogenide.

DE-B-1 210 789 describes a process for the preparation of cyano acetates, wherein a monochloroacetic acid ester is reacted with a stoichiometric excess of hydrogen cyanide in the presence of an alkali metal alcoholate in ethanol as a solvent, while the reaction mixture is heated under reflux conditions. Typically the alkali metal alcoholate is present at an alkali metal alcoholate to monochloroacetic acid ester molar ratio from 1:1.1 to 1:3. The reaction must be carried out under anhydrous conditions. The yield of the reaction is from 70 to 88% and the selectivity is from 70 to 84%.

DE-B-1 272 914 describes a process for the preparation of cyano acetates by reacting a monochloroacetic acid ester with an alkali metal cyanide and hydrogen cyanide in the presence of an inert solvent, e.g. ethanol under pressure of at least 2 bar. Again, the reaction is carried out under anhydrous conditions. The yield of the reaction is from 80 to 90% and the selectivity is from 70 to 89%.

DE-A-1 951 032 describes a process for the preparation of cyano acetates wherein a monochloroacetic acid ester is reacted with an excess of an alkali metal cyanide in aqueous acetonitrile as a solvent at atmospheric pressure. Use of hydrogen cyanide is intentionally avoided. The reaction time at 50°C is about 4 to 7 hours and the yield is from 77% to 80%.

US-A-4 322 369 describes the preparation of cyano acetates by cyanidation of a monochloroacetic acid ester with an excess of alkali cyanide and, optionally, hydrogen cyanide in an inert solvent, e.g. acetonitrile, in the presence of a sequestering agent. The sequestering agents used in this process are tertiary amines capable of forming a complex with the alkali metal cyanids, wherein this complex is more soluble in the solvent utilized than the alkali metal cyanide itself. The reaction must be carried out under strictly anhydrous conditions and reaction time is from 5 to 10 hours. The yield is from 72 to 94%.

EP-A-0 999 206 describes a process for the synthesis of cyano acetates by reacting a monochloroacetic acid ester with hydrogen cyanide in the presence of a base in an inert dried solvent. Examples for the base are tertiary amines, quaternary ammonium salts and quaternary phosphonium salts. The reaction is carried out under anhydrous conditions, and in order to achieve yields and selectivity above 90 % the base is used in amounts which are about equimolar to the monochloroacetic acid ester.

IN-A-217086 describes a process for the preparation of cyano acetates by reacting a monochloroacetic ester with alkali cyanide in the presence of a catalyst such as a quaternary ammonium salt. The reaction takes place in a biphasic reaction medium consisting of water and a water-immiscible organic solvent. The reaction time is about 4 hours and the yield is about 96%.

The processes used in the prior art have the drawback that they either require long reaction times or large amounts of a base to obtain high product yields and high selectivities. Generally, high conversion rates often result in a decreased selectivity, i.e., in the formation of more side products.

The object of the present invention, therefore, is to provide a simple process for the preparation of cyano carboxylic acid esters that allows high reaction rates and product yields thereby maintaining high selectivities, said process being also compatible with production on an industrial level.

This object has been achieved by the process of the invention for the preparation of a cyano carboxylic acid ester of the formula 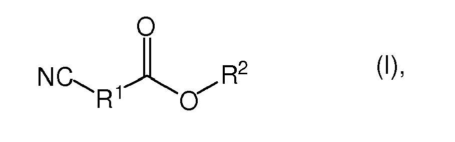 wherein
R¹ is a linear or branched C₁₋₈ alkanediyl group; and
R² is a linear or branched alkyl group, a cycloalkyl group, or an aryl or arylalkyl group,
wherein aryl is optionally substituted with one or more C₁₋₈ alkyl groups;
comprising reacting a halo carboxylic acid ester of the formula 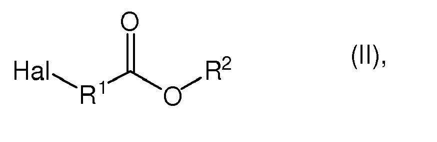 wherein
R¹ and R² are as defined above; and
Hal is fluorine, chlorine, bromine or iodine;

in a reaction mixture comprising a homogeneous liquid phase, wherein the liquid phase consists of water and an organic solvent,
with alkali metal cyanide and hydrogen cyanide, optionally in the presence of a catalyst, to obtain the cyano carboxylic acid ester of the formula (I).

It has been found that under these reaction conditions the reaction is completed within 0.5 to 1.5 hours with excellent product yield and selectivity.
(2) Hal in the compound of the formula (II) is fluorine, chlorine, bromine or iodine Preferably, Hal is bromine or chlorine, most preferably chlorine.

In the compounds of the formulas (I) and (II), R¹ is a linear or branched C₁₋₈ alkanediyl group. Examples of C₁₋₈ alkanediyl groups are methanediyl, ethane-1 ,2-diyl, propane-1,2-diyl, propane-1,3-diyl, propane-2,2-diyl, butane-1,2-diyl, butane-1,4-diyl, 2-methylpropane-1,2-diyl, 2-methylpropane-1 ,3-diyl, pentane-1,5-diyl, hexane-1,6-diyl and heptane-1,7-diyl.
(3)

Preferably, R¹ is a linear or branched C₁₋₄ alkanediyl group, more preferably a linear C₁₋₄ alkanediyl group, and most preferably a methanediyl or an ethane-1,2-diyl group.
(4)

R² in the compounds of the formulas (I) and (II) may be a linear or branched alkyl group, a cycloalkyl group, an aryl group or an aralkyl group.

Preferably, the alkyl group of R² is a linear or branched C₁₋₈ alkyl group. Examples for linear or branched C₁₋₈ alkyl groups are ethyl, propyl, isopropyl, *n-*butyl, sec-butyl, isobutyl, *tert*-butyl, *n-*pentyl, *iso*-pentyl, sec-pentyl, *neo-*pentyl, 1,2-dimethylpropyl, *n-*hexyl, *iso*-hexyl, *sec*-hexyl, *n-*heptyl, *iso*-heptyl, *n-*octyl, 2-ethylhexyl. More preferably, R² is a C₁₋₄ alkyl group, even more preferably methyl, ethyl, propyl or isopropyl. Most preferred are ethyl and propyl.

Cycloalkyl is preferably C₃₋₈ cycloalkyl, more preferred C₃₋₆ cycloalkyl. Examples for C₃₋₆ cycloalkyl are cyclopropyl, cyclobutyl, cyclopentyl or cyclohexyl.

Aryl is preferably phenyl or naphthyl, optionally substituted with one or more, preferably one or two, C₁₋₈ alkyl groups, wherein C₁₋₈ alkyl is as defined above and preferably is a linear or branched C₁₋₄ alkyl group, most preferably methyl or ethyl. Preferably, aryl is substituted or unsubstituted phenyl.

Aralkyl is an aryl substituted alkyl group, wherein alkyl is preferably a linear or branched C₁₋₈ alkyl group, more preferably a C₁₋₄ alkyl group, and aryl is preferably phenyl or naphthyl, optionally substituted with one or more, preferably one or two, C₁₋₈ alkyl groups, more preferably linear or branched C₁₋₄ alkyl groups, most preferably methyl or ethyl. Preferably, aralkyl is benzyl. (5)

Preferably, the halo carboxylic acid ester of the formula (II) is a chloroacetic acid ester or a chloropropionic acid ester, more preferably the methyl or ethyl ester thereof, for example ethyl chloroacetate or ethyl 3-chloropropionate.
(6)
Preferably, the alkali metal cyanide is used in a molar ratio of alkali metal cyanide to halo carboxylic acid ester in the range of from 1:1 to 1.5:1, more preferably of from 1.05:1 to 1.1:1. Conveniently, the alkali metal cyanide will be added to the reaction in solid form with or without residual moisture content.

The alkali metal cyanide may be lithium cyanide, sodium cyanide, potassium cyanide or a mixture thereof. Sodium cyanide is preferred.
(7)

Hydrogen cyanide in the process of the invention is preferably used in a molar ratio of hydrogen cyanide to halo carboxylic acid ester in the range of from 0.7:1 to 4:1, more preferably of from 1.5:1 to 3:1. It has been found that the presence of hydrogen cyanide increases the selectivity of the reaction due to the formation of a pH-buffer which suppresses the formation of by-products which typically occurs at higher pH-values. As the true cyanidation agent is the cyanide ion, the use of hydrogen cyanide in addition to the alkali metal cyanide to improve the selectivity does not result in a loss of educt material.
(8)

Preferably, the catalyst is used in a catalyst to halo carboxylic acid ester molar ratio of not more than 0.1:1. Preferably, the molar ratio is in the range of from 0.01:1 to 0.05:1, more preferably in the range of from 0.02:1 to 0.04:1, for example about 0.03:1.
(9)

Expediently, the catalyst used in the present invention comprises for example a quaternary ammonium salt, a quaternary phosphonium salt, a ionophor, a linear polyether or a mixture thereof, more preferably the catalyst is selected from quaternary ammonium salts, quaternary phosphonium salts and a mixture thereof.

Examples of quaternary ammonium salts and quaternary phosphonium salts are those of the formula 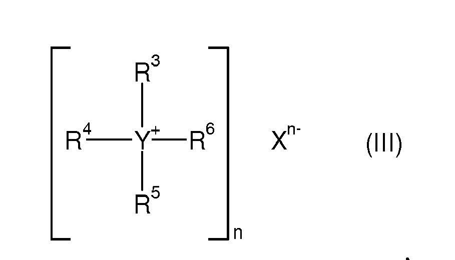 wherein z
R³, R⁴, R⁵ and R⁶ independently are a linear or branched alkyl group, a cycloalkyl group, an aryl group or an aralkyl group;
Y is P or N;
X is an inorganic anion; and
n is 1 or 2.

Preferably, the alkyl group in the definition of R³, R⁴, R⁵ and R⁶ is a linear or branched C₁₋₂₀ alkyl group. Examples for linear or branched alkyl groups are ethyl, propyl, isopropyl, *n-*butyl, sec-butyl, *iso*-butyl, *tert*-butyl, *n-*pentyl, *iso*-pentyl, sec-pentyl, *neo-*pentyl, 1,2-dimethylpropyl, *n-*hexyl, *iso*-hexyl, sec-hexyl, *n-*heptyl, *iso*-heptyl, *n-*octyl, 2-ethylhexyl, decyl, dodecyl, hexadecyl or octadecyl. More preferably, the alkyl group is a linear or branched C₁₋₁₂ alkyl group, in particular a linear or branched C₄₋₆ alkyl group, for example butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,-trimethylpropyl, 1-ethyl-1-methylpropyl and 1-ethyl-2-methylpropyl. Linear, i.e., n-alkyl groups are preferred.

Cycloalkyl, aryl and aralkyl are as defined above for R².

Y is P or N, wherein N is preferred.

Preferably, R³, R⁴, R⁵ and R⁶ independently are a linear or branched alkyl group.

The inorganic anion X is a suitable counterion. Preferably X is a halogenide, hydrogensulfate, sulfate, hydrogencarbonate, carbonate, tetrafluoroborate, trifluoromethansulfonate, acetate, perchlorate, dihydrogenphosphate, hydrogenphosphate or nitrate. More preferably X is a halogenide or carbonate, wherein the halogenide may be a fluoride, chloride, bromide or iodide. Preferred halogenides are bromide and iodide.

Ammonium and phosphonium salts useful in the present invention include, for example, quaternary tetraaryl, alkyltriaryl, dialkyldiaryl, trialkylaryl and tetraalkyl ammonium or phosphonium salts and mixtures thereof.

Examples of suitable quaternary ammonium salts are C₄₋₁₈-*n*-alkyldimethylbenzylammonium halogenides, in particular C₄₋₁₄-*n*-alkyldimethylbenzylammonium halogenides, and dimethyl(diC₄₋₁₄-*n*-alkyl)ammonium halogenides and mixtures thereof, wherein the halogenide preferably is a bromide, chloride or iodide. Specific examples of useful quaternary ammonium salts are tetramethylammonium chloride, tetramethylammonium bromide, trimethylethylammonium iodide, trimethylcetylammonium bromide, trimethylbenzylammonium chloride, dimethylethylcetylammonium chloride, dimethyloctylbenzylammonium chloride, dimethyloctadecylbenzylammonium chloride, dimethylphenylbenzylammonium bromide, dimethyldibenzylammonium bromide, methylethylpropylisobutylammonium chloride, (tetradecyl)trimethylammonium chloride, methylcetyldibenzylammonium bromide, tetrabutylammonium chloride, tetrabutylammonium bromide, tetrabutylammonium iodide, cetyldimethylethylammonium bromide, dioctyldimethylammonium chloride, dioctyldimethylammonium bromide, dioctyldimethylammonium iodide, octyldecyldimethylammonium chloride, octyldecyldimethylammonium bromide, octyldecyldimethylammonium iodide, didecyldimethylammonium chloride, didecyldimethylammonium bromide, didecyldimethylammonium iodide and mixtures thereof.

Examples of suitable quaternary phosphonium salts are tetraphenylphosphonium bromide, hexadecyltributylphosphonium bromide, tetraphenylphosphonium chloride, tetraphenylphosphonium iodide, tetrabutylphosphonium chloride, triphenylbenzylphosphonium iodide, triethylbenzylphosphonium chloride and tricyclohexylbenzylphosphonium chloride and mixtures thereof.

Ionophores are for example coronands (crowns or crown ethers), cryptands, podants and lariat ethers, or spherands.

Coronands are cyclic polyethers comprising ethylene bridged oxygen atoms. Preferably for the alkali metal ions Li+, Na+, or K+ the crown ethers 12-crown-4, 15-crown-5 or 18-crown-6, respectively, are used to form a stable salt. Further examples of crown ethers are .diaza-18-crown-6, dicyclohexane-18-crown-6, dibenzo-18-crown-6 and mixtures thereof

Cryptands are for example polyoxadiazamacrodi-, tri-, or polycyclic compounds, such as [2,2,1]cryptand (4,7,13,16,21-pentaoxa-1,10-diazabizyclo[8,8,5]-tricosan), [2,1,1]cryptand (4,7,13,18-teraoxa-1,10-diazabizyclo[8,5,5]-eicosan) or [2,2,2]cryptand (4,7,13,16,21-24-hexaoxa-1,10-diazabizyclo[8,8,8]-hexacosan). Preferably for the following alkali metal ions Li+, Na+, or K+ the kryptands [2,1,1]cryptand, [2,2,1]cryptand or [2,2,2]cryptand, respectively, are used to form a stable salt.

Podants are open chained ionophores, comprising donor atoms such as oxygen or nitrogen in at least one linear or branched chain. Podants can also be attached as side arms of crown ethers to form lariat ethers.

Li⁺ and Na⁺ can also be complexed by spherands (for example 6,12,18,24,30,36-hexamethoxy-3,9,15,21,27,33-hexamethyl[0.6]metacyclophane) built of cyclic m-phenylen units.

Examples of linear polyethers are polyetheylene glycols (PEG), preferably those having a number average molecular weight (Mₙ) of from 300 g/mol to 3000 g/mol.
(10)

In a preferred embodiment the catalyst of the above mentioned process comprises at least one quaternary ammonium salt.
(11)

Preferably the catalyst comprises a tetrabutylammonium halide, such as tetrabutylammonium bromide, tetrabutylammonium chloride, tetrabutylammonium iodide or a mixture thereof.

The reaction of the present invention is carried out in a reaction mixture comprising a homogeneous liquid phase, said liquid phase consisting of water and an organic solvent. Water and organic solvent are thus used in amounts suitable to form a homogenous liquid phase in the presence of the compounds of formula (I). Because the compound of formula (II) has similar solubility properties as the compound of formula (I), conditions to maintain said homogenous phase can be determined easily. During the reaction a salt consisting of the alkali metal halogenide is formed. Said salt usually is insoluble in the homogenous liquid phase and thus precipitates. Maintaining a homogenous liquid phase seems to facilitate precipitation of said alkali metal halogenide.
(12)

Water may preferably be present in an amount suitable to dissolve at least part of the alkali metal cyanide. Preferably, the molar ratio of water to halo carboxylic acid ester in the reaction mixture will be in the range of from 0.75:1 to 4:1, more preferably of from 1.3:1 to 3:1.

Expediently, the organic solvent is inert under the reaction conditions. Examples of solvents useful in the invention are methanol, ethanol, *n-*propanol, isopropanol, *n-*butanol, sec-butanol, isobutanol, *tert*-butanol, cyclohexanol, acetonitrile, proprionitrile, butyronitrile, ethylene glycol monoether, ethylene glycol dimethylether, dimethylformamide, dimethylsulfoxide, tetrahydrofuran, dioxane and mixtures thereof.
(13)

Acetonitrile, propionitrile, butyronitrile, methanol, ethanol and mixtures thereof are preferred solvents in the instant process, particularly preferred are acetonitrile, methanol and mixtures thereof.

The organic solvent is used in such amounts that in the reaction mixture a homogeneous liquid phase is formed with the water which contains the dissolved alkali metal cyanide. Typically, the organic solvent is used in a molar ratio of solvent to halo carboxylic acid ester in the range of from 0.5:1 to 10:1, more preferably in a molar ratio in the range of from 1:1 to 6:1. Although higher amounts of organic solvent are possible, such higher amounts might not have a positive effect on the reaction rate.

The process of the invention is typically carried out by mixing a halo carboxylic acid ester, an alkali metal cyanide, optionally a catalyst, and an organic solvent in a reaction vessel followed by the addition of hydrogen cyanide, preferably in gaseous or liquid form. The cyanidation reaction is started by the addition of water which dissolves the solid alkali metal cyanide. Alternatively, hydrogen cyanide and water can be added together in the form of an aqueous solution of hydrogen cyanide.
(14)

The reaction may be carried out over a wide temperature range. Preferably, the reaction is carried out at a temperature of from 10 to 90 °C, preferably of from 20 to 80 °C, more preferably of from 40 to 75°C. The reaction is typically performed at atmospheric pressure.
(15)

In a preferred embodiment of the invention, the hydrogen cyanide is recycled after the reaction is completed. After filtration and separation of the reaction mixture from the product, for example by distillation, the reaction mixture can be reused as such, optionally accomplished by water, solvent or HCN. Recycling of HCN is possible after separating HCN by distillation, removal from the reaction mixture by bubbling with an inert gas such as nitrogen. It is also possible to hydrolyse the amount of HCN which is necessary in the next reaction cycle by the addition of a base. In that case the organic solvent should be inert toward basic hydrolysis, such as for example methanol, ethanol, *n-*propanol, isopropanol, *n-*butanol, sec-butanol, isobutanol, *tert*-butanol, cyclohexanol, ethylene glycol monoether, ethylene glycol dimethylether, dimethylformamide, dimethylsulfoxide, tetrahydrofuran, dioxane and mixtures thereof.. Two or more of the above mentioned methods might be used in combination to recycle HCN.

The reaction is usually allowed to proceed for 0.5 to 2.5 hours, more preferred for 0.5 to 1.5 hours. Usually, the reaction is essentially completed within a period of from about 0.5 to 1.5 hours.

With the process of the present invention excellent yields and selectivities could be achieved in a very short reaction time. The reaction can be carried out in homogenous phase in a simple and convenient manner as no anhydrous conditions are required and the optional catalyst can be used in small amounts. The good results obtained in a homogeneous reaction phase are unexpected, as catalysts usually require a biphasic system to function properly. The reaction in homogeneous phase in the presence of an alkali metal cyanide and hydrogen cyanide can be carried out even without a catalyst to yield good results. The presence of hydrogen cyanide allows the formation of a buffer which increases the selectivity of the reaction as the buffer system suppresses the formation of by-products. The hydrogen cyanide may be recycled after completion of the reaction so that no educt material is lost. The process of the invention may further be carried out under energetically favourable conditions as no elevated pressure or low temperature is required.

The present invention is now illustrated in more detail with reference to the following Examples which do not limit the scope of the invention.

### Examples

### Preparation of ethyl cyanoacetate

### Example 1

1 eq of ethyl chloroacetate, 1 eq of sodium cyanide (NaCN), 0.03 eq of tetrabutylammonium bromide (TBAB) and 4 eq of acetonitrile were mixed in an agitator at room temperature followed by the addition of 1.55 eq of gaseous hydrogen cyanide (HCN). 2.25 eq of water were added and the obtained mixture was kept at a temperature of 55 °C for 2 hours. The reaction was cooled to room temperature and the mixture was subjected to filtration to remove the formed NaCl. A sample was taken and the content of ethyl cyanoacetate and of residual ethyl chloroacetate was determined by gas chromatography (GC) to calculate conversion rate, yield and selectivity. The results are shown in Table 1 below. Before or after separation of the salt, the product can be isolated according to known techniques for example by distillation or chromatography such as HPLC or gel permeation chromatography.

### Examples 2 to 32

Examples 2 to 32 were carried out as described in Example 1 using ethyl chloroacetate (1 eq) and NaCN (1 eq) but with different amounts of TBAB, water and HCN at various temperatures. The amounts used and the results achieved are summarized in Table 1 below.

As can be seen in Table 1 below, conversion rates were consistently above 90% with selectivities of more than 97%.

**Table 1**

| Example No. | Reaction temp. [°C] | NaCN [eq] | TBAB [eq] | HCN [eq] | H₂O [eq] | Conversion [%] | Yield [%] | Selectivity [%] |
|---|---|---|---|---|---|---|---|---|
| 1 | 55 | 1.00 | 0.03 | 1.55 | 2.26 | 94.2 | 93.2 | 99.0 |
| 2 | 45 | 1.00 | 0.04 | 1.50 | 2.25 | 94.2 | 92.3 | 98.0 |
| 3 | 55 | 1.00 | 0.04 | 1.48 | 2.95 | 94.7 | 92.3 | 97.5 |
| 4 | 65 | 1.00 | 0.03 | 1.50 | 1.57 | 96.9 | 94.9 | 97.9 |
| 5 | 55 | 1.00 | 0.01 | 2.23 | 2.26 | 93.0 | 90.6 | 97.5 |
| 6 | 45 | 1.00 | 0.03 | 2.22 | 2.30 | 93.0 | 92.0 | 98.9 |
| 7 | 45 | 1.00 | 0.03 | 0.75 | 2.25 | 95.1 | 94.1 | 99.0 |
| 8 | 55 | 1.00 | 0.03 | 0.74 | 1.57 | 96.4 | 93.8 | 97.4 |
| 9 | 65 | 1.00 | 0.01 | 1.46 | 2.25 | 94.4 | 92.7 | 98.2 |
| 10 | 55 | 1.00 | 0.03 | 1.46 | 2.25 | 94.6 | 92.9 | 98.2 |
| 11 | 55 | 1.00 | 0.04 | 0.74 | 2.25 | 94.6 | 92.2 | 97.5 |
| 12 | 65 | 1.00 | 0.03 | 0.71 | 2.26 | 95.9 | 93.1 | 97.1 |
| 13 | 55 | 1.00 | 0.01 | 1.48 | 1.57 | 95.4 | 93.6 | 98.1 |
| 14 | 55 | 1.00 | 0.04 | 2.27 | 2.26 | 94.2 | 93.2 | 98.9 |
| 15 | 55 | 1.00 | 0.03 | 2.23 | 1.57 | 95.2 | 93.4 | 98.1 |
| 16 | 45 | 1.00 | 0.03 | 1.46 | 2.93 | 92.1 | 89.8 | 97.5 |
| 17 | 55 | 1.00 | 0.01 | 1.50 | 2.93 | 92.1 | 90.3 | 98.1 |
| 18 | 55 | 1.00 | 0.03 | 2.22 | 2.93 | 93.7 | 92.0 | 98.2 |
| 19 | 55 | 1.00 | 0.03 | 1.48 | 2.26 | 94.0 | 92.1 | 98.0 |
| 20 | 65 | 1.00 | 0.03 | 1.46 | 2.93 | 94.6 | 92.0 | 97.3 |
| 21 | 65 | 1.00 | 0.04 | 1.45 | 2.26 | 96.1 | 93.5 | 97.3 |
| 22 | 55 | 1.00 | 0.04 | 1.46 | 1.57 | 96.7 | 94.4 | 97.6 |
| 23 | 65 | 1.00 | 0.03 | 2.23 | 2.25 | 93.2 | 93.2 | 99.9 |
| 24 | 55 | 1.00 | 0.03 | 0.73 | 2.93 | 92.7 | 89.6 | 96.7 |
| 25 | 45 | 1.00 | 0.03 | 1.47 | 1.57 | 94.0 | 92.2 | 98.1 |
| 26 | 55 | 1.00 | 0.03 | 1.48 | 2.25 | 94.2 | 91.9 | 97.5 |
| 27 | 50 | 1.00 | 0.025 | 1.00 | 2.25 | 95.7 | 91.9 | 96.0 |
| 28 | 50 | 1.00 | 0.025 | 1.00 | 2.25 | 98.1 | 93.4 | 95.2 |
| 29 | 50 | 1.10 | 0.025 | 1.50 | 2.25 | 98.2 | 94.5 | 96.2 |
| 30 | 50 | 1.10 | 0.025 | 1.50 | 1.80 | 97.8 | 94.7 | 96.6 |
| 31 | 50 | 1.05 | 0.025 | 1.50 | 2.25 | 96.8 | 93.3 | 96.4 |
| 32 | 50 | 1.05 | 0.013 | 1.50 | 2.25 | 94.3 | 90.6 | 96.1 |

### Preparation of ethyl cyanoacetate with and without catalysts

### Example 33

Ethyl chloroacetate (1 eq), sodium cyanide (NaCN, 1.05 eq), tetrabutylammonium bromide (TBAB, 0.025 eq) and acetonitrile (10 eq) were mixed in an agitator at room temperature followed by the addition of gaseous hydrogen cyanide (HCN, 1.5 eq). Water (2.25 eq) was added and the obtained mixture was kept at a temperature of 50 °C for 2 hours. Samples were taken at different points of time and yield was determined by gas chromatography (GC), wherein T = 0 is the first measurement after complete addition of water. The results are shown in Table 2 below.

### Example 34

The reaction was carried out as described in Example 33 except that 0.025 eq of tetrabutylammonium iodide (TBAI) was used as a catalyst. The results are shown in Table 2 below.

### Comparative Example 1

The reaction was carried out as described in Example 33 except that 0.025 eq of NaBr was used instead of a catalyst. The results are shown in Table 2 below.

**Table 2**

| Example No. | 33 | 34 | Comp. example 1 |
|---|---|---|---|
| Reaction time [min] | Catalyst | | |
| | TBAB | TBAI | NaBr |
| | Yield [%] | | |
| 0 | 65.7 | 63.8 | 42.4 |
| 15 | 82.5 | 83.5 | 58.0 |
| 30 | 91.0 | 92.4 | 73.6 |
| 60 | 94.3 | 96.0 | 85.2 |
| 90 | 95.1 | 96.2 | 89.6 |
| 120 | 95.1 | 96.8 | 92.6 |

As will be seen from the above results, in the presence of a catalyst yield of ethyl cyanoacetate was more than 90% within 30 min, while yield was only about 74% in the absence of a catalyst. After 1 hour, the reaction was essentially completed in the presence of TBAB and TBAI. In the presence of NaBr, yield reached about 93% only after 2 hours, which is still below the yield obtained in the presence of TBAB and TBAI. The high yield corresponds to a high selectivity at high conversion rates.

### Preparation of ethyl cyanoacetate with different amounts of organic solvent

### Examples 35 to 37

The reaction was carried out as described in Example 33 except that different amounts of acetonitrile were used as an organic solvent. The results are shown in Table 3 below.

**Table 3**

| Example No. | 35 | 36 | 37 |
|---|---|---|---|
| Reaction time [min] | Acetonitrile [eq] | | |
| | 10 | 8 | 6 |
| | Yield [%] | | |
| 0 | 65.7 | 55.8 | 61.7 |
| 15 | 82.5 | 76.0 | 82.3 |
| 30 | 91.0 | 86.3 | 88.1 |
| 60 | 94.3 | 91.9 | 92.7 |
| 90 | 95.1 | 94.3 | 93.9 |
| 120 | 95.1 | 94.9 | 94.6 |

As will be seen from the above results, the amount of solvent had no significant effect on the yield in ethyl cyanoacetate.

### Examples 38 and 39

Ethyl chloroacetate (80 g, 1 eq), sodium cyanide (32 g, 1 eq) and acetonitrile (160 g, 10 eq) were mixed in an agitator at room temperature followed by the addition of gaseous hydrogen cyanide (39.7 g, 2.25 eq). Water (18.5 g, 1.75 eq) was added and the obtained mixture was kept at a temperature of 65 °C for 2 hours. The experiment was carried out twice. After workup according to example 1, conversion rates of 94.7 and 94.4%, yields of 92.6 and 92.0%, and selectivities of 97.8 and 97.3%, were obtained, respectively.

## Claims

1. A process for the preparation of a cyano carboxylic acid ester of the formula 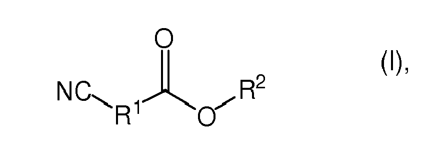 wherein
R¹ is a linear or branched C₁₋₈ alkanediyl group; and
R² is a linear or branched alkyl group, a cycloalkyl group, or an aryl or arylalkyl group, wherein aryl is optionally substituted with one or more C₁₋₈ alkyl groups;
comprising reacting a halo carboxylic acid ester of the formula 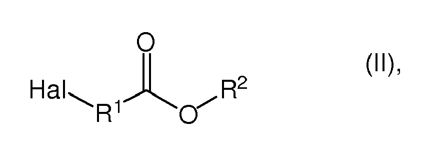 wherein
R¹ and R² are as defined above; and
Hal is fluorine, chlorine, bromine or iodine;
in a reaction mixture comprising a homogeneous liquid phase, wherein the liquid phase consists of water and an organic solvent,
with an alkali metal cyanide in the presence of hydrogen cyanide, optionally in the presence of a catalyst,
to obtain the cyano carboxylic acid ester of the formula (I).

2. The process of claim 1, wherein Hal in the compound of the formula (II) is selected from fluorine, chlorine and bromine, preferably is chlorine or bromine.

3. The process of claims 1 or 2, wherein R¹ is a linear or branched C₁₋₄ alkanediyl group, more preferably a linear C₁₋₄ alkanediyl group.

4. The process of any one of claims 1 to 3, wherein R² is a linear C₁₋₄ alkyl group.

5. The process of any one of claims 1 to 4, wherein the compound of the formula II is selected from chloroacetic acid esters or chloropropionic acid esters, preferably is ethyl chloroacetate or ethyl 3-chloropropionate.

6. The process of any one of claims 1 to 5, wherein the alkali metal cyanide is used in a molar ratio of alkali metal cyanide to halo carboxylic acid ester in the range of from 1:1 to 1.5:1.

7. The process according to any of claims 1 to 6, wherein the hydrogen cyanide is used in a molar ratio of hydrogen cyanide to halo carboxylic acid ester in the range of from 0.7:1 to 4:1.

8. The process of any one of claims 1 to 7, wherein the catalyst is used in a catalyst to halo carboxylic acid ester molar ratio of not more than 0.1 to 1, preferably in the range of from 0.01:1 to 0.05:1.

9. The process of any one of claims 1 to 8, wherein the catalyst is selected from the group consisting of quaternary ammonium salts, quaternary phosphonium salts, ionophores, linear polyethers and mixtures thereof.

10. The process of any one of claims 1 to 7, wherein the catalyst comprises at least one quaternary ammonium salt.

11. The process of claim 8, wherein the quaternary ammonium salt is a tetrabutylammonium halide.

12. The process according to any of claims 1 to 11, wherein water is used in a molar ratio of water to halo carboxylic acid ester in the range of from 0.75:1 to 4:1.

13. The process of any one of claims 1 to 12, wherein the organic solvent is selected from the group consisting of acetonitrile, propionitrile, butyronitrile, methanol, ethanol and mixtures thereof.

14. The process of any one of claims 1 to 13, wherein the reaction is carried out at a temperature of from 10 to 90 °C.

15. The process of any one of claims 1 to 14, wherein the hydrogen cyanide is recycled at the end of the reaction.
